# EUROPEAN PATENT SPECIFICATION

(11) **EP 2 227 163 B1**
(45) Date of publication and mention of the grant of the patent: **10.10.2012**
(21) Application number: 08857858.8
(22) Date of filing: 21.11.2008
(51) Int. Cl.: A61B 18/14

(54) **IMPLANTABLE DEVICE WITH ELECTROLYTICALLY DETACHABLE JUNCTION HAVING MULTIPLE FINE WIRES**
IMPLANTIERBARE VORRICHTUNG MIT ELEKTROLYTISCH ABLÖSBARER VERBINDUNG MIT MEHREREN FEINEN DRÄHTEN
DISPOSITIF IMPLANTABLE AVEC UNE JONCTION DÉTACHABLE DE FAÇON ÉLECTROLYTIQUE AYANT DE MULTIPLES CÂBLES MINCES

(30) Priority: 03.12.2007 US 991856 P
(43) Date of publication of application: 15.09.2010
(73) Proprietor: Stryker Corporation, Kalamazoo, MI 49002 (US); Stryker NV Operations Limited, Dublin 2 (IE)
(72) Inventor: BASHIRI, Mehran, San Carlos California 94070 (US); CHU, Stella, Fremont California 94538 (US); CHANG, Esther, Fremont California 94536 (US)
(74) Representative: Hauck Patent- und Rechtsanwälte
(86) International application number: PCT/US2008/084298
(87) International publication number: WO 2009/073398

(56) References cited:
- EP-A- 0 803 230
- EP-A- 0 804 905
- EP-A- 1 323 385
- EP-A- 1 329 196
- US-A- 5 947 962
- US-A- 5 947 963
- US-A1- 2002 151 883
- US-A1- 2004 181 215

## Description

### FIELD OF THE INVENTION

The invention generally relates to implantable devices and, more particularly, to a temporary, electrolytically detachable junction members.

### BACKGROUND

Implants such as vaso-occlusive have been used in various applications including treatment of intra-vascular aneurysms. One known vaso-occlusive device is a soft, helically wound coil. One known coil is formed by winding a platinum wire strand about a primary mandrel and applying heat to the mandrel to impart a primary winding coil shape. The device is then wrapped around a secondary mandrel, and heat is applied to the secondary mandrel to impart a secondary shape. U.S. Patent Nos. 4,994,069 and 5,354,295 provide examples of known vaso-occlusive coils and methods of deploying coils to treat aneurysms.

U.S. Patent No. 5,354,295 describes an embolism forming device and procedure employing an electrolytically severable joint. A platinum coil is delivered to a vascular cavity, such as an aneurysm, using a catheter and a deployment mechanism, such as a pusher or core wire, which has a stainless steel coil or joint attached to the distal end thereof. After the vaso-occlusive coil in its primary shape is placed in the aneurysm, a small electrical current is applied to the core wire to form a clot, which forms a thrombus or collagenous mass that contains the vaso-occlusive device therein. The vaso-occlusive coil is detached from the core wire by electrolysis, in which electrical current applied to the core wire dissolves the stainless steel coil or joint that is exposed to blood and attached to the distal end of the core wire, thereby detaching the vaso-occlusive coil at the aneurysm site. The core wire and catheter can then be retracted, leaving the vaso-occlusive coil in the aneurysm.

### SUMMARY

In accordance with one embodiment, an assembly includes an implantable device, a conductive deployment mechanism and an electrolytically detachable junction member between the deployment mechanism and the implantable device. The deployment mechanism is used to deliver to the implantable device to a desired location. The electrolytically detachable junction member includes a plurality of fine wires that extend between the implantable device and the conductive deployment mechanism so that when electrical current is applied to the fine wires through the conductive deployment mechanism, the fine wires are electrolyzed and detached from the implantable device.

In accordance with another embodiment, an assembly includes an implantable vaso-occlusive coil, a conductive deployment mechanism and an electrolytically detachable junction member. The deployment mechanism is used to deliver the vaso-occlusive coil to a desired location. The electrolytically detachable junction member includes a plurality of fine stainless steel wires that have a diameter of about 12.7 µm (0.0005") so that when electrical current is applied to the fine stainless steel wires through the conductive deployment mechanism, the fine stainless steel wires are electrolyzed and detached from the vaso-occlusive coil.

In accordance with a further alternative embodiment, an assembly includes an implantable vaso-occlusive coil, a conductive deployment and an electrolytically detachable junction member. The electrolytically detachable junction member includes a plurality of fine stainless steel wires having a diameter of about 12.7 µm (0.0005") and a length of about 25.4 µm (0.01"). Each fine stainless steel wire is partially coated with non-conductive coating. A first, proximal portion of each wire is bare. A second portion adjacent the first portion is coated with the non-conductive coating. A third portion adjacent the second portion is bare. A fourth, distal portion adjacent the third portion is coated with the non-conductive coating. The deployment mechanism is used to deliver the vaso-occlusive coil to a desired location. When electrical current is applied to proximal end of the fine stainless steel wires through the conductive deployment mechanism, the wires are electrolyzed and detached from the vaso-occlusive coil.

In various embodiments, junction member can include wires made of materials, such as stainless steel, that can be electrolyzed. The junction member can include different numbers of wires, e.g., about two to ten wires and larger numbers of wires, e.g. as many as 500 or more fine wires. All of the wires can have the same diameter or different diameters. For example, a junction member may include about 20 wires, and the diameter of some or all of the wires can be about 12.7 µm (0.0005") A cross-sectional surface area of the group of fine wires may be about 25.35cm² (3.93 x 10⁻⁶ inch²). For a 21.54 µm (0.010") length detachment zone including about 20 fine wires, the cylindrical surface area for all of the fine wires can be about 20,26.10⁻⁴ (3.14 x 10⁻⁴ inch²). The fine wires can extend freely between the deployment mechanism and the implantable device or they can be wound or braided. The fine wires can also be partially coated for connecting to the deployment mechanism and the implantable device. For example, proximal ends of the fine wires can be bare and connected by a conductive connection, such as solder or a conductive polymer, to the conductive deployment mechanism. This allows current to flow through the deployment mechanism and to the fine wires. Distal ends of the fine wires can be covered with a non-conductive coating and inserted into a non-conductive barrier member, such as a polymer barrier member, of the implantable device.

### BRIEF DESCRIPTION OF THE DRAWINGS

Referring now to the drawings in which like reference numbers represent corresponding parts throughout:
Figure 1 generally illustrates one embodiment of an assembly having an electrolytically detachable junction having a plurality of fine wires that join a deployment mechanism and an implantable device;
Figure 2 illustrates an electrolytically detachable junction having two fine wires according to one embodiment;
Figure 3 illustrates an electrolytically detachable junction having two fine wires and connected to a deployment mechanism and an implantable device using solder according to one embodiment;
Figure 4 illustrates an electrolytically detachable junction having two fine wires and welded to a deployment mechanism and an implantable device according to one embodiment;
Figure 5 illustrates an electrolytically detachable junction having two fine wires and welded to a deployment mechanism and an implantable device according to one embodiment;
Figure 6 is a view of the embodiment shown in Figures 4 and 5 along lines A-A;
Figure 7 generally illustrates fine wires having various bends and curves;
Figure 8 generally illustrates one embodiment of an electrolytically detachable junction having more than two fine wires;
Figure 9 is a view of the embodiment shown in Figure 8 along lines B-B;
Figure 10 generally illustrates one embodiment of an electrolytically detachable junction having multiple fine wires that are wound or braided according to one embodiment;
Figure 11 is a view of the embodiment shown in Figure 10 along lines C-C;
Figure 12 generally illustrates an electrolytically detachable junction having fine wires of different sizes that are wound or braided according to one embodiment;
Figure 13 is a view of the embodiment shown in Figure 12 along lines D-D;
Figure 14 illustrates a fine wire that is partially coated according to one embodiment;
Figure 15 illustrates an assembly according to one embodiment that includes an electrolytically detachable junction having two fine wires extending between a pusher wire and a vaso-occlusive coil;
Figure 16 further illustrates an assembly according to one embodiment that includes an electrolytically detachable junction having two fine wires and a vaso-occlusive coil;
Figure 17 illustrates another assembly according to one embodiment that includes an electrolytically detachable junction having two fine wires and a vaso-occlusive coil;
Figure 18 illustrates an assembly according to one embodiment that includes an electrolytically detachable junction having fine wires that are wound or braided and a vaso-occlusive coil;
Figure 19 is a flow chart of a procedure including delivering and detaching an implantable device using an electrolytically detachable junction having fine wires, shown for the purpose of better understanding the invention;
Figure 20 generally depicts how embodiments can be used in assemblies that deliver and implant a vaso-occlusive coil in a cranial aneurysm;
Figure 21 generally depicts the assembly shown in Figure 20 after the electrolytically junction having fine wires has been electrolyzed to detach the vaso-occlusive coil in the aneurysm; and
Figure 22 is a table that demonstrates dimensions of various embodiments.

### DETAILED DESCRIPTION OF ILLUSTRATED EMBODIMENTS

In the following description, reference is made to the accompanying drawings which form a part hereof, and which show by way of illustration specific embodiments in which the invention may be practiced.

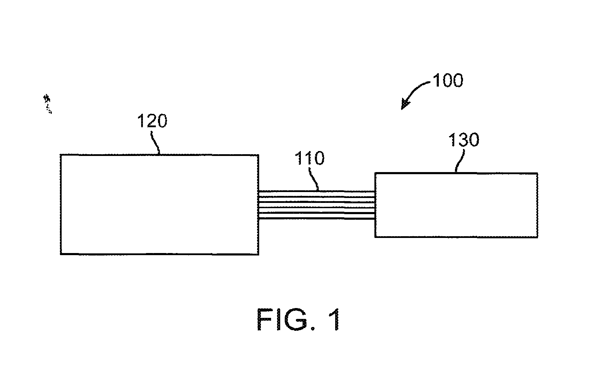

Referring to Figure 1, one embodiment is directed to an assembly 100 that includes an electrolytically detachable junction member 110, which is a temporary connection or sacrificial link including a plurality of fine or small diameter wires. The fine wires join a conductive deployment mechanism 120, such as a wire, pusher, etc., and an implantable device 130, such as a vaso-occlusive coil. The deployment mechanism 120 is used to deliver the implantable device 130 to a treatment site, such as a cranial aneurysm. During use, after the device 130 is properly positioned, electrical current from a current source external to the body is applied to the conductive deployment mechanism 120 and through the fine wire junction member 110 resulting in formation of a thrombus at the treatment site. The electrical current simultaneously electrolyzes and disintegrates the fine wires of the junction member 110, thereby detaching the implantable device 130 and leaving the device 130 at the treatment site and in the thrombus.

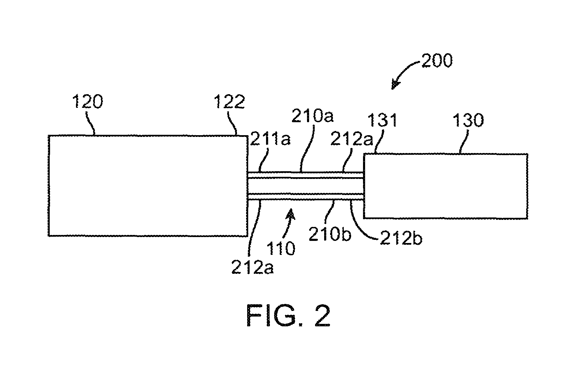

Referring to Figure 2, an assembly 200 constructed according to one embodiment includes a junction member 110 having two fine wires 210a and 210b (generally referred to as fine wires 210) that extend between the deployment mechanism 120 and the implantable device 130. According to one embodiment, the fine wires 210 are stainless steel. In an alternative embodiment, the fine wires 210 are made of tungsten or other suitable materials.

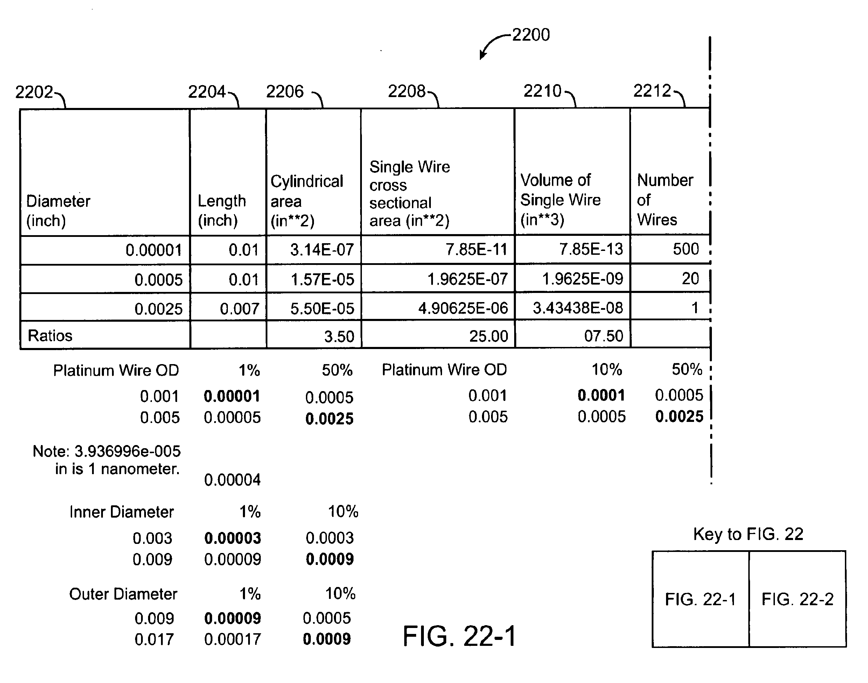

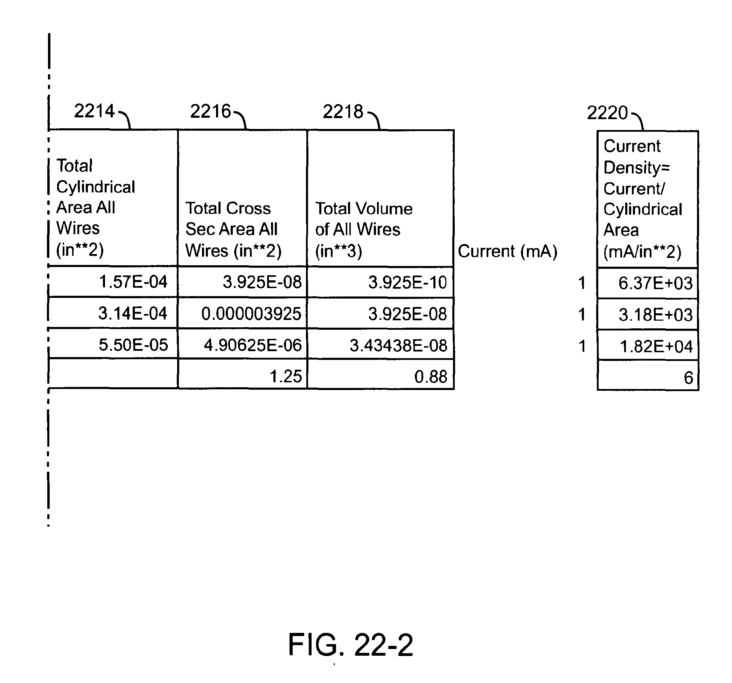

As used in this specification, a "fine" wire 210 is defined as a wire having a small diameter, i.e., about 0.254 µm (0.00001") to about 63,5 µm (0.0025") for example, about 12,7µm (0.0005"). According to on embodiment, such fine wires are stainless steel wires. According to one embodiment, a cross sectional area of an individual fine wire 210 is about 50,65 cm². 10⁻¹⁰ (7.85 x 10⁻¹⁰ inch²) to about 31,62.10⁻⁵ cm² (4.9 x 10⁻⁵ inch²) and a volume of a single wire may be about 20,65.10⁻³ cm² (7.85 x 10⁻¹³) to about 22,13.10⁻⁸ cm² (3.43 x 10⁻⁸) inch³). A length of a fine wire may be about 2.54 µm (0.01") to about 17.72 µm (0.0007") and a length of an etched area of a fine wire 210 can be, for example, about 25,4µm (0.001") to about 2,54mm (0.100"). Fine wire 210 dimensions may vary depending on the length of the fine wire 210. For example, longer fine wires 210 may have a larger diameter. In one embodiment, a fine wire 210 having a length of about 2.54µm (0.01") may have a diameter of about 2.54µm (0.0001") to about 12.7µm (0.0005") and a fine wire 210 having a length of about 7.78 µm (0.007") may have a diameter of about 63,5 µm (0.0025"). This specification refers to fine stainless steel fine wires 210 having diameters ranging from about 0,254 µm (0.00001") to about 63,5 µm (0.0025") for ease of explanation, and it should be understood that the relative dimensions of the assembly components shown in the figures are not necessarily representative of actual devices since relative component sizes are adjusted for purposes of illustration (e.g., as shown in Figure 22).

With continuing reference to Figure 2, the proximal ends 211 a, 211b (generally 211) of respective fine wires 210a, 210b are attached to the distal end 122 of the deployment mechanism 120. The distal ends 212a, 212b (generally 212) of the fine wires 210a, 210b are attached to the proximal end 131 of the implantable device 130. The proximal ends 211 of the fine wires 210 may be joined to the distal end 122 of the deployment mechanism 120 using various known methods and materials.

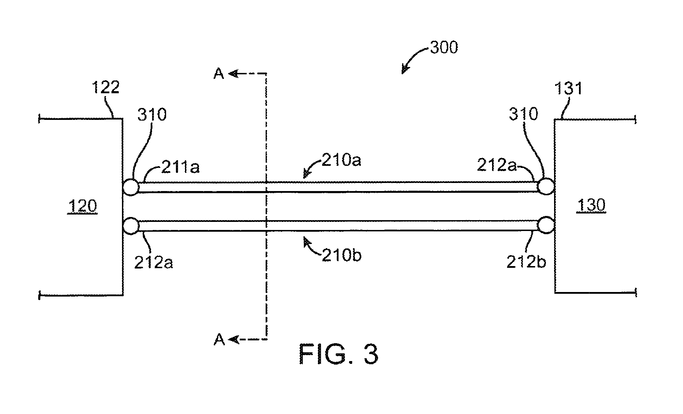

Referring to Figure 3, in an assembly 300 constructed according to one embodiment, fine wires 210 are joined to the conductive deployment mechanism 120 using solder, a conductive polymer, such as conductive polyacetylene, and other suitable conductive materials 310. For ease of explanation, this specification generally refers to solder 310, but embodiments may be implemented with other conductive materials that may attach fine wires 210 to the deployment mechanism 120.

Proximal ends 211 of the fine wires 210 may contact the solder 310 as in the embodiment shown in Figure 3, or may be embedded within the solder 310. In the embodiment illustrated in Figure 3, proximal ends 211 of the fine wires 210 are connected to the distal end 122 of the deployment mechanism 120 using balls of solder 310. It should be understood that solder balls 310 may be spherical and other shapes after the proximal ends 211 are joined to and/or embedded in the solder 310. Figure 3 also illustrates that distal ends 212 of the fine wires 210 may be joined to a proximal end 131 of the implantable device 130 by soldering.

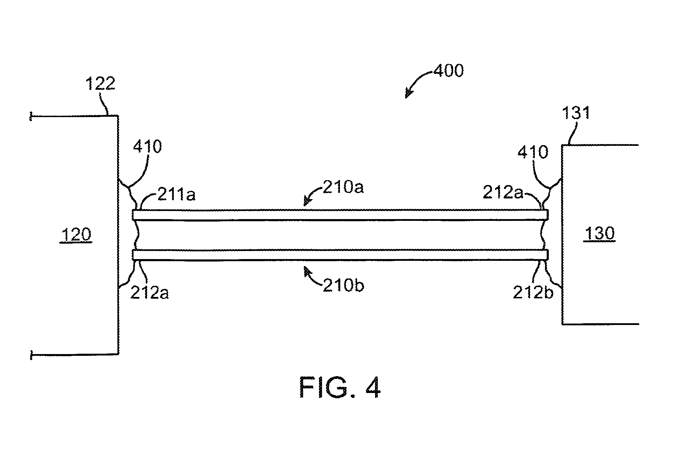

Referring to Figure 4, in an assembly 400 constructed according to an alternative embodiment, the proximal ends 211 of the fine wires 210 may be welded 410 to the distal end 122 of the deployment mechanism 120. Figure 4 illustrates the proximal ends 211 a, 212a of both fine wires 210a, 210b being welded 410 to the deployment mechanism 120 using a single portion of welding material or a single weld. In an alternative embodiment, the fine wires 210a, 210b may be separately welded. Figure 4 also illustrates that the distal ends 211 b, 212b of the fine wires 210a, 210b may be welded 410 to a proximal end 131 of the implantable device 130.

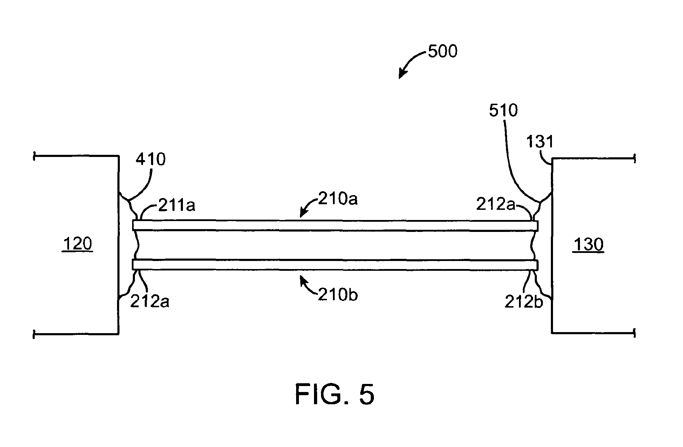

Referring to Figure 5, in an assembly 500 constructed according to another alternative embodiment, rather than attaching the distal ends 212a, 212b of the fine wires 210a, 210b to the proximal end 131 of the implantable device 130 by conductive connections, such as soldering 310 or welding 410, the distal ends 212a, 212b may be attached or inserted into a non-conductive material, layer or barrier 510, such as a non-conductive polymer or glue. The non-conductive polymer material 510 isolates the fine wires 210 from the implantable device 130. Thus, various Figures illustrates that different ends of the fine wires 210 can be attached to different components using different materials.

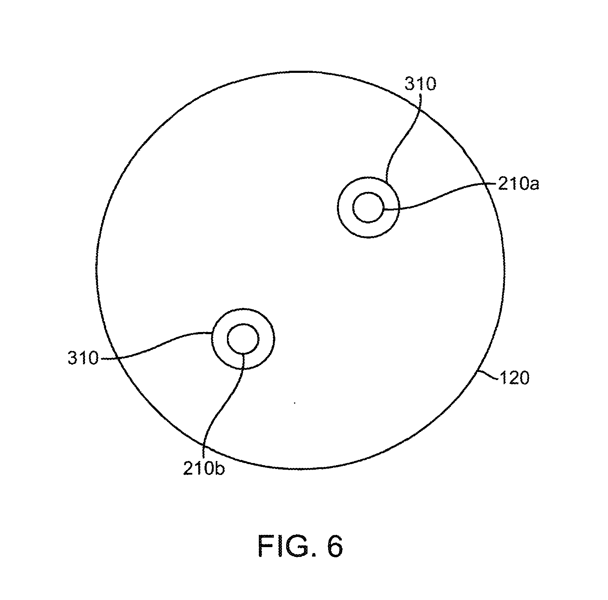

Figure 6 illustrates a view along line A-A shown in Figure 3 and illustrates that the fine wires 210 may be horizontally and/or vertically offset (as illustrated) or they can be aligned vertically or aligned horizontally (not shown in Figure 6). In the illustrated embodiment, the proximal end of one fine wire 210a is connected to an upper right portion of the distal end 122 of the deployment mechanism 120, and another fine wire 21 0b is connected to a lower left portion of the distal end 122 of the deployment mechanism 120. The fine wires 210 can also be connected to other parts of the deployment mechanism 120 as necessary. Additionally, it should be understood that the cross-sectional shape of the deployment mechanism 120 can be circular (as shown in Figure 5) or other shapes. Further, although Figure 6 illustrates an embodiment in which the fine wires 210a, 210b are connected to the distal end 122 of the deployment mechanism 120 with solder (as shown in Figure 3), the connection may be formed using other types of materials (as discussed with reference to Figures 4 and 5).

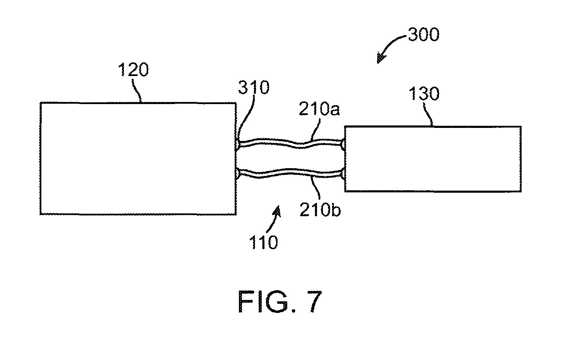

Figures 1-6 illustrate embodiments in which an electrolytically detachable junction member 110 includes two straight, fine wires 210a, 210b. During use, the fine wires 210 may include various bends and curves, e.g., as shown in Figure 7. Thus, Figures that show straight fine wires 210 are not intended to be limiting since the fine wires 210 are flexible and bendable.

Additionally, Figures 1-7 illustrate embodiments in which the electrolytically detachable junction member 110 includes two fine wires 210a, 210b. In alternative embodiments, the junction member 110 may include more than two fine wires 210, e.g., three fine wires, five fine wires, ten fine wires, and other numbers of fine wires 210 as needed.

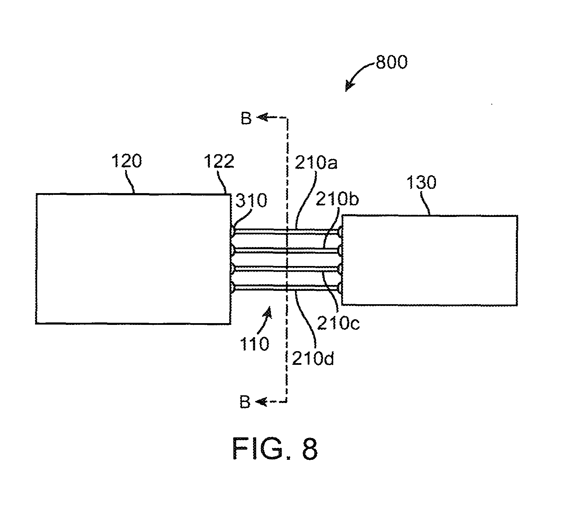

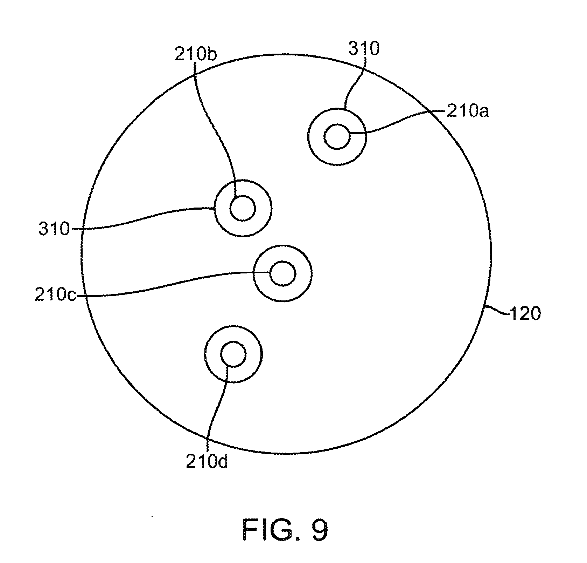

For example, in the embodiment shown in Figures 8 and 9, an assembly 800 constructed according to another embodiment includes an electrolytically detachable junction member 110 having four fine wires 210a-d. Figure 8 also shows separate conductive joining elements 310, e.g., separate solder balls, for joining a proximal end 211 of each fine wire 210 or a group of fine wires 210 to the distal end 122 of the deployment mechanism 120.

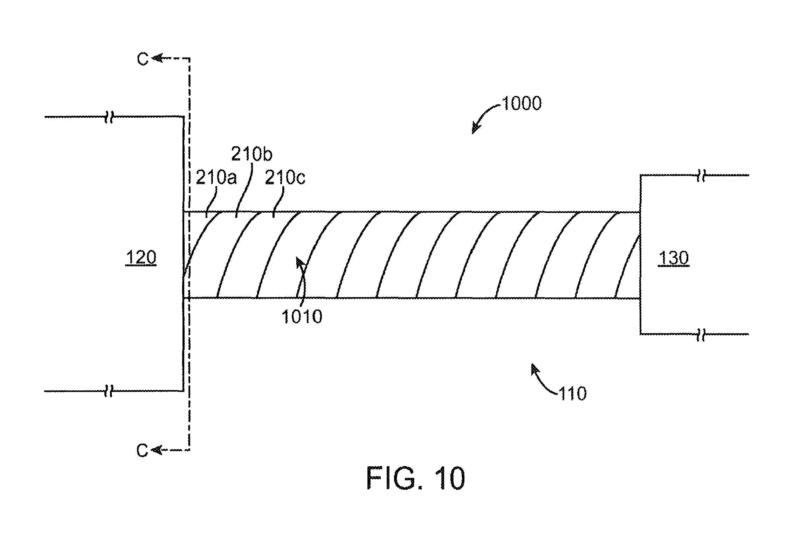

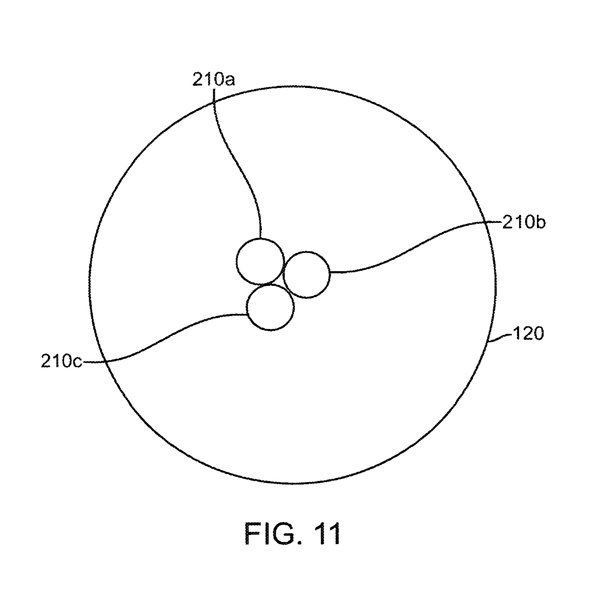

Referring to Figures 10 and 11, in one embodiment, the electrolytically detachable junction member 110 may include multiple fine wires 210 that are wound or braided together 1010. According to one embodiment as shown in Figures 10 and 11, the wound fine wires 210 have the same diameter. This configuration may be particularly useful for increasing the strength and flexibility of the junction member 110. In the illustrated embodiment, the assembly 1000 includes three fine wires 210a-c that are braided or wound 1010 around each other. The connections at the proximal ends 211 and distal ends 212 of the fine wires 210 are omitted for clarity. In the illustrated embodiment, three fine wires 210 are wound or braided 1010, however, other numbers of fine wires 210 may be wound or braided 1010, including two, four, ten or other numbers of fine wires 210 as necessary.

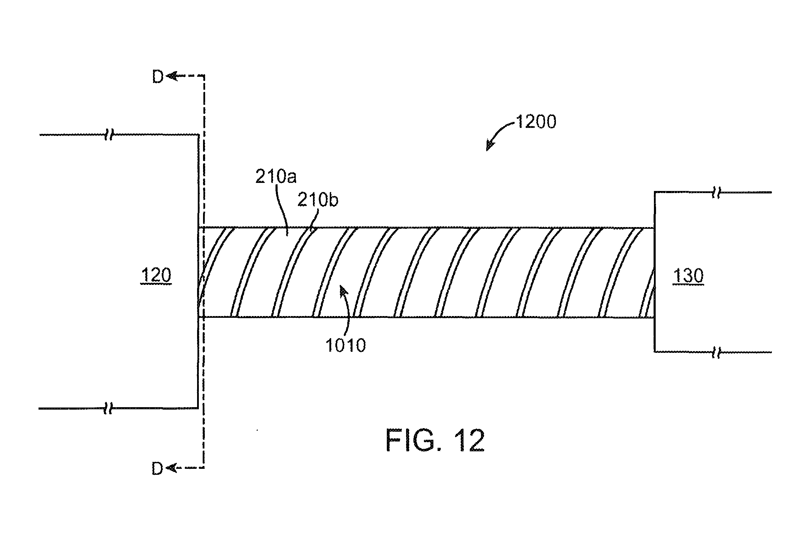

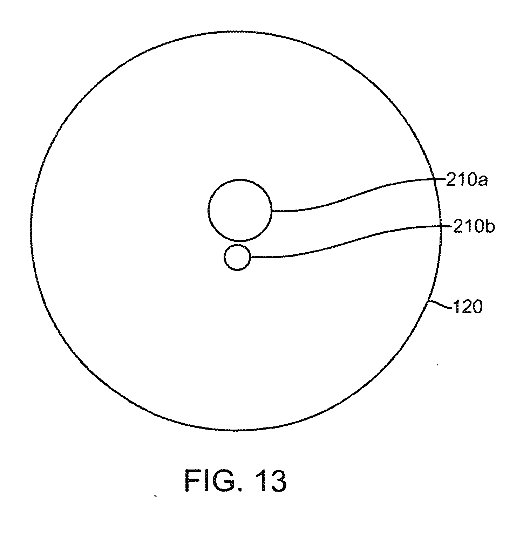

Referring to Figures 12 and 13, an assembly 1200 constructed according to an alternative embodiment includes fine wires 210a and 210b that have different diameters and are wound or braided 1010 around each other. The connections at the proximal ends 211 and distal ends 212 of the fine wires 210 are again omitted for clarity. In other embodiments, different numbers of fine wires 210 having different diameters can be wound or braided 1010 together. Thus, in embodiments, there may be one or more fine wires 210 of a first diameter and one or more wires 210 of a second diameter. There may also be one or more fine wires 210 of different numbers of diameters, e.g., one or more fine wires 210 of a first diameter, one or more fine wires 210 of a second diameter, and one or more fine wires 210 of a third diameter. Accordingly, Figure 12 and 13 are provided to illustrate different manners in which embodiments may be implemented to form a wound or braided 1010 junction member including fine wires 210 of different diameters.

Further, it should be understood that with respect to embodiments shown in Figures 1-13, the number and arrangement of fine wires 210 may depend on, for example, the fine wire 210 material, length, diameter(s), connections, whether the fine wires extend freely between the deployment mechanism and the implantable device or whether they are wound or braided and the desired tensile strength, and desired current density of each wire 210.

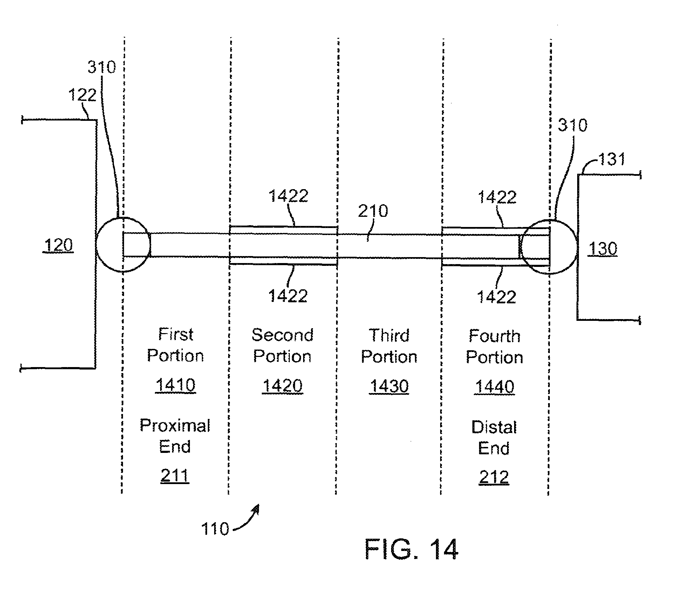

Referring to Figure 14, according to another embodiment, fine wires 210 of the junction member 110 may be partially covered to define conductive and non-conductive sections and attachment points or zones. For ease of explanation and for purposes of illustration, Figure 14 illustrates a single fine wire 210 that extends between a deployment mechanism 120 and an implantable device 130, but it should be understood that embodiments may involve various numbers of fine wires 210 that may be coated in a similar manner.

In the illustrated embodiment, the fine wire 210 is intermittently or alternately coated. In the illustrated embodiment, a first portion 1410 of the fine wire 210 at the proximal end 211 of the wire 210 is bare or uncoated, a second portion 1420 adjacent to the first portion 1410 is coated with a non-conductive coating 1422, a third portion 1430 adjacent to the second portion 1420 is bare, and a fourth portion 1440 at the distal end 212 of the wire 210 is coated with a non-conductive coating 1442, which can be the same as or different than the non-conductive coating 1422 of the second portion 1420. The non-conductive coatings 1422 and 1442 can be, for example, a polymide polymer or Parylene.

In the illustrated embodiment, the first portion 1410 is bare and connected to the deployment mechanism 120 with solder 310 or another suitable conductive connection, to allow conduction of electrical current from the conductive deployment mechanism 120, through the fine wire 210 and to the implantable device 130. The non-conductive coatings 1422 and 1442 of the second and fourth portions 1420 and 1440 define a bare third portion 1430, which is a detachment point or zone. Thus, the fourth portion 1440 includes a non-conductive coating 1442 to define the detachment zone (as previously discussed) and to facilitate connection to the non-conductive polymer or glue 500 that is applied to the proximal end 131 of the implantable device 130. With this configuration, during use, the assembly is delivered to a treatment site, and the third portion 1430 is exposed to blood, which is conductive. Electrical current is applied to conductive deployment mechanism 120 and to the fine wire 210, thereby causing electrolysis and disintegration of the third portion 1430 and detachment of the implantable device 130 at the treatment site.

Figure 14 shows the four different portions 1410, 1420, 1430, 1440 having similar dimensions. However, the portions may have different lengths. For example, the bare first portion 1410 can have a length that is about 1% to about 50% of the length of a fine wire 210, the coated second portion 1420 can have a length that is about 5% to about 90% of the length of a fine wire 210, the bare third portion 1430 can have a length that is about 1% to about 50% of the length of a fine wire 210, and the coated fourth portion 1440 can have a length that is about 5% to about 90% of the length of a fine wire 210.

Further, the bare third portion 1430 may be shorter than other portions to define a particular detachment point or zone. The length of the first portion 1410 may be shorter depending on how much of the fine wire 210 is connected to the distal end 122 of the deployment mechanism 120. For example, what may be required is a short length of the bare first portion 1410, e.g., a first portion 1410 that is about 2mm long, or about 20% of a length of a fine wire 210, to allow the conductive distal end 211 to be connected to the conductive deployment mechanism 120 via the conductive solder 310, weld 410 or polymer. Accordingly, it should be understood that different portions can have the same or similar length and different lengths as necessary.

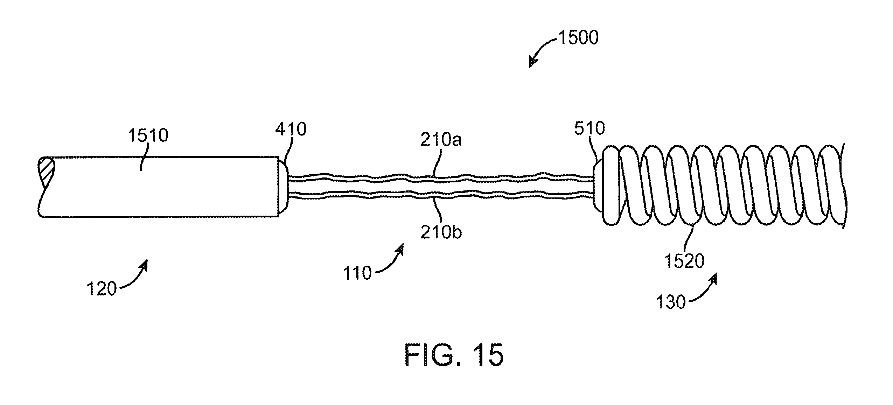

Referring to Figure 15, an assembly 1500 constructed according to one embodiment includes a deployment mechanism 120 that is a wire or pusher 1510, an electrolytically detachable junction 110 that includes two fine wires 210a, 210b, and an implantable device 130 in the form of a vaso-occlusive coil 1520, such as the vaso-occlusive coil described in U.S. Patent Nos. 5,112,136 and 5,354,295. The vaso-occlusive coil 1520 shown in Figure 15 is illustrated as assuming a primary shape, i.e., a linear helical shape, when being introduced through a delivery catheter.

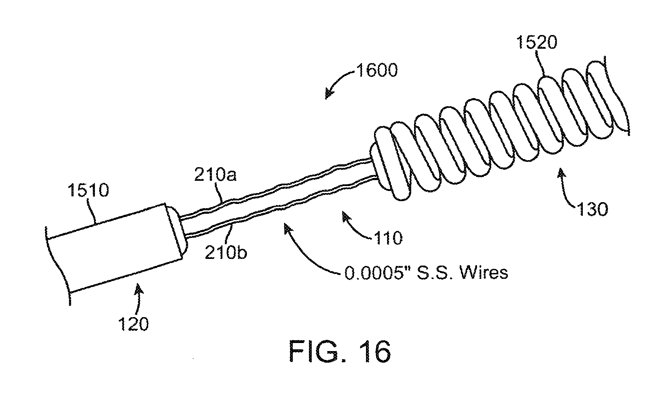

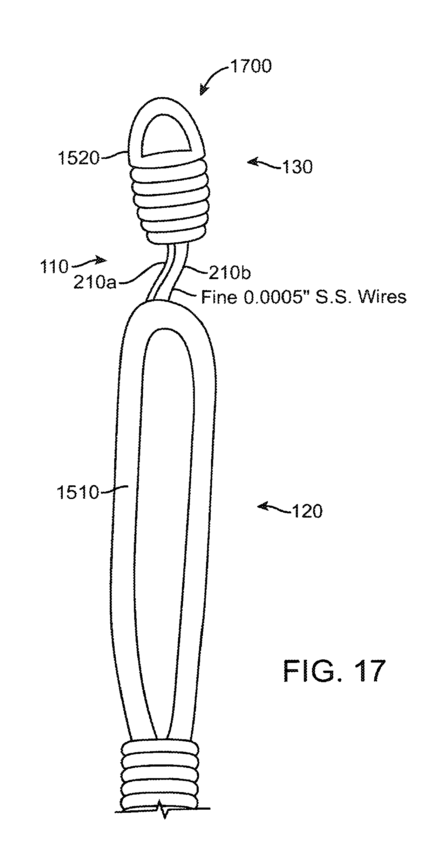

Figures 16 and 17 further illustrate embodiments of assemblies 1600 and 1700 that were made using different types of wires or pushers 1510, an electrolytically detachable junction 110 and a vaso-occlusive coil 1520. In these embodiments, the fine wires 210 had a diameter of about 12.7µm (0.0005").

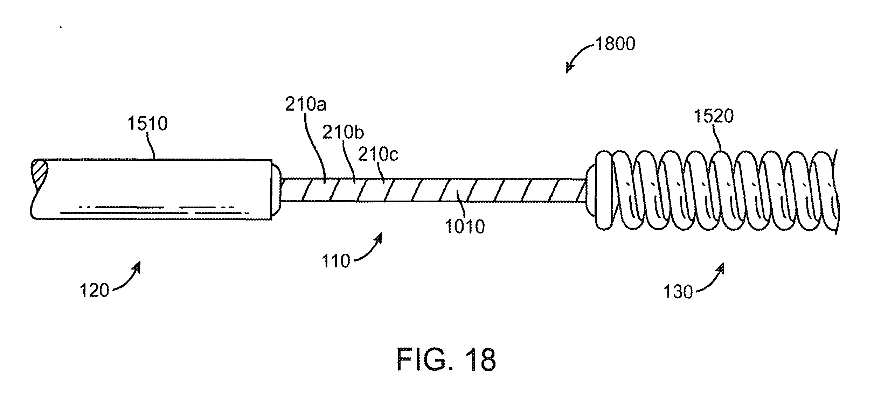

Additionally, referring to Figure 18, an assembly 1800 constructed according to another embodiment includes multiple fine wires 210a-c that are wound or braided 1010 and a vaso-occlusive coil 1520 (e.g., as discussed above with reference to Figures 10-13).

A typical vaso-occlusive coil 1520 is formed of platinum wire having a diameter of about 25.4 µm (0.001") to about 1.27µm (0.005"). The primary helical shape has an inner diameter of about 76,2µm (0.003") to about 228,6µm (0.009") and the outer diameter of the primary shape is about 228.6µm (0.009") to about 431.8µm (0.017") According to one embodiment, the diameter of a stainless steel fine wire 210 is about 10-50% of the diameter of the platinum wire that is used to form the vaso-occlusive coil. Further, according to another embodiment, the diameter of each fine wire 210 is about 1% to about 10% of an inner diameter of a primary shape of a wound implantable vaso-occlusive coil. Thus, with a primary shape having an inner diameter of about 76.2 µm (0.003") to about 22.86 µm (0.0009"), the diameter of a fine wire 210 may be about 0.762 µm (0.0003") to about 22.86 µm (0.0009") Further, according to a further embodiment, the diameter of each fine wire 210 is about 1% to about 5% of an outer diameter of a primary shape of a wound implantable vaso-occlusive coil. Thus, with a primary shape having an outer diameter of about 22.86 µm (0.009") to about 431.8 µm (0.017") the diameter of a fine wire 210 may be about 2.29 µm (0.00009") to about 21.76 µm (0.0009").

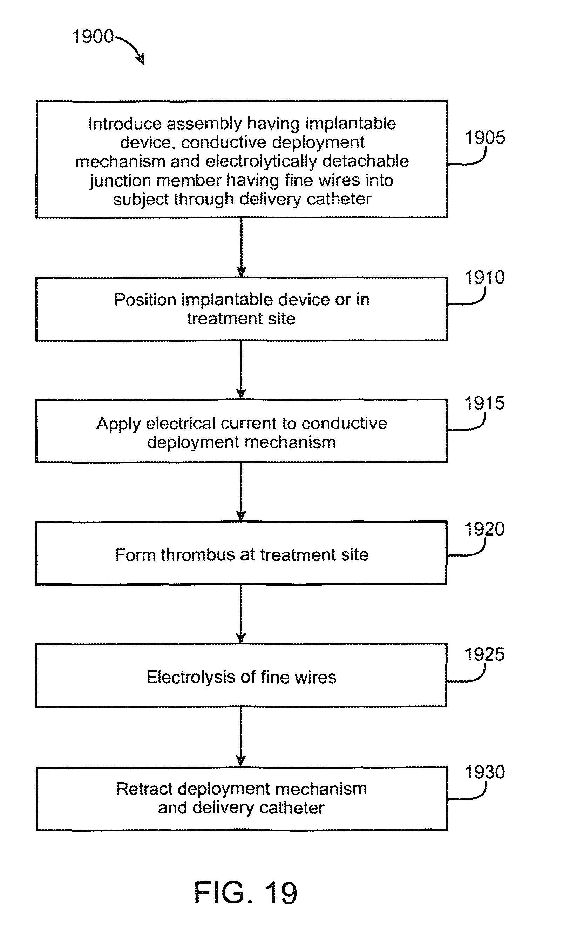

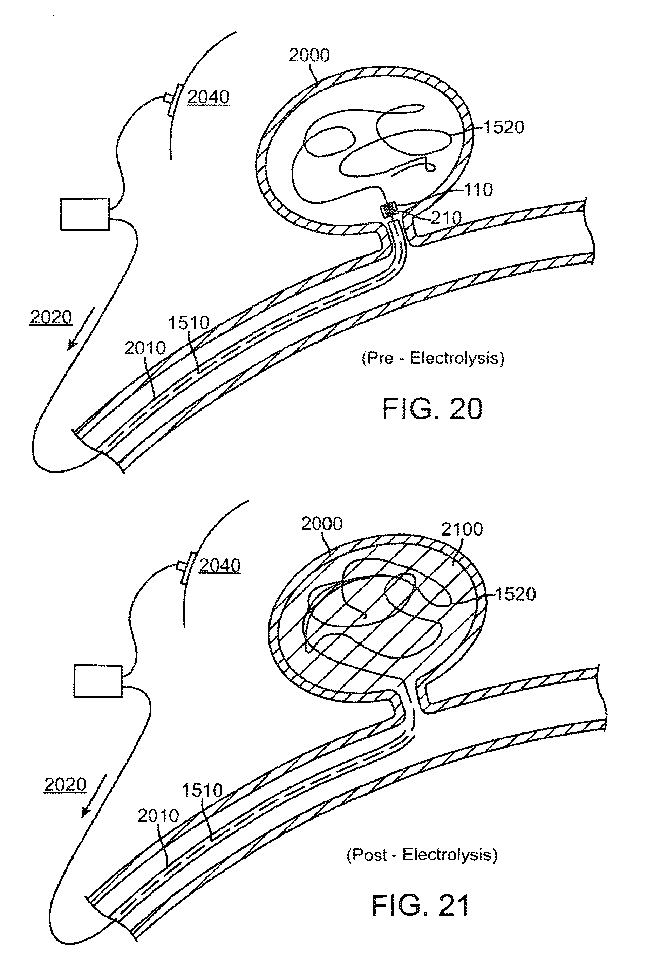

Referring to Figures 19-21, for purposes of better understanding the invention, a procedure 1900 including introducing an implantable device, such as a vaso-occlusive coil 1520, into a subject or patient to treat an aneurysm 2000 includes introducing an assembly having a vaso-occlusive coil 1520, a conductive deployment mechanism 120, such as a wire or pusher 1510, and electrolytically detachable junction member 110 having fine wires 210 into a patient in step 1905. This can be done by introducing the assembly into the patient through a delivery catheter 2010. In step 1910, the wire or pusher 1510 is manipulated to position the coil 1520 at or in the aneurysm 2000. Thus, as shown in Figure 20, the distal end of the delivery catheter 2010 is maneuvered into the neck of the aneurysm 2000. As shown in Figure 20, the coil 1520 assumes a primary or linear helical shape when it is introduced through the delivery catheter 1510, and upon exiting the delivery catheter 2010, the coil 1520 assumes a relaxed, convoluted shape inside the aneurysm 2000. In step 1915, after the coil 1520 is positioned and deployed in the aneurysm 2000, electrical current 2020 is applied from a current source 2030 external to the body 2040 and to the conductive wire 1510. Electrical current 2020 is conducted through the wire 1510 and through the fine wires 210 of the electrolytically detachable junction 110 and to the coil 1520.

As a result, in step 1920, and as shown in Figure 21, a thrombus 2100 is formed in the aneurysm 2000 following application of electrical current 2020. In step 1925, after the thrombus 2100 is formed, the fine wires 210 of the detachable junction 110 are disintegrated, as shown in Figure 21, thereby detaching the coil 1520 in the aneurysm 2000. More particularly, the portions of the fine wires 210 that are bare and exposed to blood are disintegrated. Thus, with reference to Figure 14, for example, the bare third portion 1430 of each fine wire 210 is disintegrated, thereby defining a detachment point or zone. Continuing with step 1930, the wire 1510 and the delivery catheter 2010 can be retracted, thereby leaving the coil 1520 in the aneurysm 2000. Further details concerning electrolytic detachment are provided in U.S. Patent No. 5,354,295.

Figures 19-21 show delivery of a vaso-occlusive coil 1520, but the implantable member 130 can be other implantable devices 130, including a filter, such as a filter to capture embolic debris, and a stent, such as a self expanding stent, a balloon expanding stent, a coated or non-coated stent, a covered or partially covered stent, a high density brain stent or a stent covered in-situ etc. Further, embodiments can be used to deliver an implantable device, including occlusive devices, in various vascular cavities including arteries, veins, aneurysms, vascular malformations, and arteriovenous fistulas.

Embodiments including fine wire 210 electrolytically detachable junction members 110 provide a number of improvements and advantages over known electrolytically detachable junction members. For example, with embodiments, the surface area of the junction member 110 is substantially increased relative to the surface area of known junction members as a result of the junction member 110 including multiple fine wires 120. Increasing junction member 110 surface areas advantageously results in reduced current densities on the junction member 110. Reduced current densities achieved with embodiments advantageously result in fewer detachment byproducts being formed and deposited and more efficient detachment processes. Further, increased surface areas achieved with embodiments allow the same or similar current densities to be maintained while increasing overall current per mass, which improves corrosion.

For example, a cylindrical surface area of a detachment zone of a known electrolytically detachable device is about 35.48.10⁻⁵ cm² (5.5 x 10⁻⁵ inch²), and the cylindrical surface area of a detachment zone of an electrolytically detachable junction member 110 including fine wires 210 constructed according to embodiments can be about 20.26.10⁻⁷ cm² (3.14 x 10⁻⁷ inch²) to about 1.29.10⁻⁵ cm² (1.75 x 10⁻⁵ inch²) e.g., with about 1 to about 500 fine wires 210 or more fine wires as necessary. As a further example, the current density on known electrolytically detachable junction members is about 3.1.10³ mA/cm² (2 x 10⁴ mA/inch²) whereas with embodiments, the current density of the group of fine wires 120 is less than 2.07.10³ mA/cm² (2 x 10⁴ mA/inch²) e.g., about 1.03.10³ mA/cm² (1 x 10² mA/inch²) to about 2.07.10³ mA/cm² (2 x 10⁴ mA/inch²)

An additional improvement is that the plurality of fine wires 120 results in lower detachable junction 110 impedance, which advantageously allows for use of lower voltages. For example, the average impedance of known electrolytically detachable junction members is about 6 kOhm, and the voltage applied to a known junction member is about 6 Volts, In contrast, electrolytically detachable junction members 110 including fine wires 210 according to embodiments are capable of lower impedances and lower voltages. For example, fine wire 210 detachable junctions 110 constructed according to embodiments may have impedances less than 6 kOhm, e.g., as low as 1 kOhm, and voltages less than 6 volts, e.g., as low as 1 volt, when 1 mA of current is applied. The impedance and voltage may also vary depending on the junction configuration, and the impedance may be between about 1 kOhm and 10 kOhm, and the voltage may be between about 1 volt and about 10 volts when about 1 mA of current is applied.

Embodiments provide further improvements and advantages with these capabilities since lower voltages result in generation of less noise compared to known higher voltage devices. Further, with less variable and lower voltage conditions achieved with embodiments, changes in impedance that are reflected in voltage values are more pronounced and identifiable, thereby facilitating detection of detachment and reducing the likelihood detachment detection errors.

Further benefits that are achieved are improved strength and flexibility. The number of fine wires 120 can be selected to provide the desired strength (e.g., tensile strength) and flexibility. Embodiments provide additional support when the implantable device 130 is pushed and pulled through a catheter. Further, the fine wires 120 can be braided or wound to provide greater strength and additional support as necessary. Thus, embodiments advantageously provide detachable junctions or temporary connections that may be designed to accommodate different flexibility and tensile strength requirements while reducing current densities on the junction.

The implantable device 130 can also include radiopaque, physiologically compatible material. For instance, the material may be platinum, gold, tungsten, or alloys of these. Certain polymers are also suitable for use in the implants, either alone or in conjunction with metallic markers providing radiopacity. These materials are chosen so that the procedure of locating the implant within the vessel may be viewed using radiography. However, it is also contemplated that the implantable device may be made of various other biologically inert polymers or of carbon fiber.

The fine wires 210 having small diameters of about 0.254µm (0.00001") to about 63.5µm (0.0025") may have various lengths, cross-sectional areas, surface areas and volumes and may form cylindrical or other shape structures having various dimensions and volumes. Figure 22 is a table 2200 that includes fine wire 210 dimensions of three different embodiments (represented by rows of the table 2200).

In the chart 2200, column 2202 represents a diameter of a single fine wire 210. In the illustrated embodiments, the fine wire 210 diameter is 0.254 µm (0.00001") 12.7 µm (0.0005") and 63.5 µm (0.0025") Column 2204 represents a length of a single fine wire 210. In the illustrated embodiments, the length of the 0.254 µm (0.00001") and 12.7 µm (0.0005") diameter fine wires 210 is 2.54 µm (0.01"), and the length of the 63.5 µm (0.0025") diameter fine wire 210 is 177.80 µm (0.007"). Column 2206 represents a cylindrical area or surface area of a single fine wire 210, column 2208 represents a cross-sectional area of a single fine wire 210, and column 2210 represents a volume of a single fine wire 210. Column 2212 represents a number of fine wires 2212 that may be utilized to form a junction member 110. In the illustrated embodiments, one junction member 110 includes 500 fine wires, another junction member 110 includes 20 fine wires, and a further junction member 110 includes a single fine wire 210. Column 2214 represents a total cylindrical or surface area of a collection or group of fine wires 210, column 2216 represents a cross-sectional area of a collection of fine wires 210 and column 2218 represents a volume of the fine wire 210. Column 2220 represents current densities achieved with different embodiments.

The table 210 also indicates the range of values for different dimensions, areas and volumes, expressed as a ratio. Thus, Figure 22 illustrates that embodiments may include different numbers of fine wires 210, and that the fine wires 210 may have different dimensions. Further, the table 2200 includes other calculations and data, e.g., fine wire 210 dimensions relative to inner and outer diameters of a primary shape of a vaso-occlusive coil 1520

## Claims

1. An assembly (100, 200, 300, 400, 500, 800, 1200, 1500, 1700, 1800) comprising:
an implantable device (130, 1520);
a conductive deployment mechanism (120, 1510) for delivering the implantable device; and
an electrolytically detachable junction (110) that couple the implantable device (130, 1520) to the conductive deployment mechanism (120, 1510), **characterized in that** the electrolytically detachable junction (110) comprises a plurality of fine wires (210, 210a, 210b, 210c, 210d) being detachable from the implantable device (130, 1520) by transmission of electrical current (2020) there through.

2. The assembly (100, 200, 300, 400, 500, 800, 1200, 1500, 1700, 1800) of claim 1, the fine wires (210, 210a, 210b, 210c, 210d) configured for being simultaneously electrolyzed by an electrical current (2020) applied to the conductive deployment mechanism (120, 1510).

3. The assembly (100, 200, 300, 400, 500, 800, 1200, 1500, 1700, 1800) of claim 1 or 2, the fine wires (210, 210a, 210b, 210c, 210d) comprising stainless steel.

4. The assembly (100, 200, 300, 400, 500, 800, 1200, 1500, 1700, 1800) of any of claims 1-3, the plurality of fine wires (210, 210a, 210b, 210c, 210d) comprising two to ten fine wires.

5. The assembly (100, 200, 300, 400, 500, 800, 1200, 1500, 1700, 1800) of any of claims 1-4, each of the fine wires (210, 210a, 210b, 210c, 210d) having a diameter between 0.0000254 cm (0.00001 inch) to 0.002286 cm (0.0009inch).

6. The assembly (100, 200, 300, 400, 500, 800, 1200, 1500, 1700, 1800) of any of claims 1-4, each of the fine wires (210, 210a, 210b, 210c, 210d) having a surface area between 5.03square mm (.00000000078 square inch) to 0.00323 square mm (.000005 square inch).

7. The assembly (100, 200, 300, 400, 500, 800, 1200, 1500, 1700, 1800) of any of claims 1-6, the electrolytically detachable junction (110) comprising a first plurality of fine wires (210) each having a first diameter, and a second plurality of fine wires (210) each having a second diameter different than the first diameter.

8. The assembly (100, 200, 300, 400, 500, 800, 1200, 1500, 1700, 1800) of any of claims 1-7, the fine wires (210, 210a, 210b, 210c, 210d) being wound or braided (1010).

9. The assembly (100, 200, 300, 400, 500, 800, 1200, 1500, 1700, 1800) of any of claims 1-8, the fine wires (210, 210a, 210b, 210c, 210d) connected to the conductive deployment mechanism (120, 1510) by a solder (310) or a conductive polymer, and the fine wires (210, 210a, 210b, 210c, 210d) connected to the implantable device (130, 1520) by a non-conductive polymer.

10. The assembly (100, 200, 300, 400, 500, 800, 1200, 1500, 1700, 1800) of any of claims 1-9, wherein for each of the fine wires(210, 210a, 210b, 210c, 210d),
a first, proximal portion (1410) of the wire is bare,
a second portion (1420) adjacent the first portion (1410) is coated with a non-conductive coating (1422),
a third portion (1430) adjacent the second portion (1420) is bare, and
a fourth, distal portion (1440) adjacent the third portion (1430) is coated with a non-conductive coating (1422).

11. The assembly (100, 200, 300, 400, 500, 800, 1200, 1500, 1700, 1800) of claim 10, wherein the respective proximal portions of the fine wires (210, 210a, 210b, 210c, 210d) are in electrical contact with the conductive deployment mechanism (120, 1510), and the respective distal portions of the fine wires (210, 210a, 210b, 210c, 210d) are disposed within a non-conductive barrier between the electrolytically detachable junction (110) and the implantable device (130, 1520).

12. The assembly (100, 200, 300, 400, 500, 800, 1200, 1500, 1700, 1800) of any of claims 1-4, wherein a respective diameter of each of the fine wires (210, 210a, 210b, 210c, 210d) is between 10% to 50% of a diameter of a platinum wire used to form the implantable device(130, 1520).

13. The assembly (100, 200, 300, 400, 500, 800, 1200, 1500, 1700, 1800) of claim 12, a diameter of each fine wire being between 0.000254 cm (0.0001 inch) to 0.00635 cm (0.0025 inch), and a diameter of the platinum, wire forming the implantable device (130, 1520) being between 0.00254 cm (0.001 inch) to 0.0127 cm (0.005 inch).

14. The assembly (100, 200, 300, 400, 500, 800, 1200, 1500, 1700, 1800) of claim 12, the implantable device (130) comprising a vaso-occlusive coil (1520), and wherein a diameter of each fine wire is between 1% to 10% of an inner diameter of a primary shape of the coil (1520), and wherein a diameter of each fine wire is between 1% to 5% of an outer diameter of a primary shape of the coil (1520).

## Patentansprüche

1. Baugruppe (100, 200, 300, 400, 500, 800, 1200,1500, 1700, 1800), umfassend:
eine implantierbare Vorrichtung (130, 1520);
einen leitfähigen Einsetzmechanismus (120, 1510) zum Einbringen der implantierbaren Vorrichtung; und
eine elektrolytisch ablösbare Verbindung (110), die die implantierbare Vorrichtung (130, 1520) an den leitfähigen Einsetzmechanismus (120,1510) koppelt, **dadurch gekennzeichnet, dass** die elektrolytisch ablösbare Verbindung (110) mehrere feine Drähte (210, 210a, 210b, 210c, 210d) umfasst, die von der implantierbaren Vorrichtung (130,1520) durch Übertragen eines elektrischen Stroms (2020) durch dieselbe ablösbar sind.

2. Baugruppe (100, 200, 300, 400, 500, 800, 1200, 1500, 1700, 1800) nach Anspruch 1, wobei die feinen Drähte (210, 210a, 210b, 210c, 210d) ausgestaltet sind, gleichzeitig durch einen elektrischen Strom (2020) elektrolysiert zu werden, der an den leitfähigen Einsetzmechanismus (120, 1510) angelegt wird.

3. Baugruppe (100, 200, 300, 400, 500, 800, 1200, 1500, 1700, 1800) nach Anspruch 1 oder Anspruch 2, wobei die feinen Drähte (210, 210a, 210b, 210c, 210d) rostfreien Stahl umfassen.

4. Baugruppe (100, 200, 300, 400, 500, 800, 1200, 1500, 1700, 1800) nach einem der Ansprüche 1 bis 3, wobei die mehreren feinen Drähte (210, 210a, 210b, 210c, 210d) zwei bis zehn feine Drähte umfassen.

5. Baugruppe (100, 200, 300, 400, 500, 800, 1200, 1500, 1700, 1800) nach einem der Ansprüche 1 bis 4, wobei jeder der feinen Drähte (210, 210a, 210b, 210c, 210d) einen Durchmesser zwischen 0,0000254 cm (0,00001 Zoll) und 0,002286 cm (0,0009 Zoll) aufweist.

6. Baugruppe (100, 200, 300, 400, 500, 800, 1200, 1500, 1700, 1800) nach einem der Ansprüche 1 bis 4, wobei jeder der feinen Drähte (210, 210a, 210b, 210c, 210d) einen Oberflächenbereich zwischen 5,03 mm² (.00000000078 Quadratzoll) und 0,00323 mm² (.000005 Quadratzoll) aufweist.

7. Baugruppe (100, 200, 300, 400, 500, 800, 1200, 1500, 1700, 1800) nach einem der Ansprüche 1 bis 6, wobei die elektrolytisch ablösbare Verbindung (110) eine erste Mehrzahl feiner Drähte (210) umfasst, die jeweils einen ersten Durchmesser aufweisen, und eine zweite Mehrzahl feiner Drähte (210), die jeweils einen zweiten, vom ersten Durchmesser verschiedenen Durchmesser aufweisen.

8. Baugruppe (100, 200, 300, 400, 500, 800, 1200, 1500, 1700, 1800) nach einem der Ansprüche 1 bis 7, wobei die feinen Drähte (210, 210a, 210b, 210c, 210d) gewickelt oder geflochten (1010) sind.

9. Baugruppe (100, 200, 300, 400, 500, 800, 1200, 1500, 1700, 1800) nach einem der Ansprüche 1 bis 8, wobei die feinen Drähte (210, 210a, 210b, 210c, 210d) mit dem leitfähigen Einsetzmechanismus (120,1510) durch ein Lötmittel (310) oder ein leitfähiges Polymer verbunden sind und die feinen Drähte (210, 210a, 210b, 210c, 210d) mit der implantierbaren Vorrichtung (130, 1520) durch ein nicht-leitfähiges Polymer verbunden sind.

10. Baugruppe (100, 200, 300, 400, 500, 800, 1200, 1500, 1700, 1800) nach einem der Ansprüche 1 bis 9, wobei bei jedem der feinen Drähte (210, 210a, 210b, 210c, 210d) folgendes zutrifft:
ein erster, proximaler Abschnitt (1410) des Drahtes liegt frei,
ein zweiter Abschnitt (1420), der dem ersten Abschnitt (1410) benachbart ist, ist mit einer nicht-leitfähigen Beschichtung (1422) beschichtet,
ein dritter Abschnitt (1430), der dem zweiten Abschnitt (1420) benachbart ist, liegt frei, und
ein vierter, distaler Abschnitt (1440), der dem dritten Abschnitt (1430) benachbart ist, ist mit einer nicht-leitfähigen Beschichtung (1422) beschichtet.

11. Baugruppe (100, 200, 300, 400, 500, 800, 1200, 1500, 1700, 1800) nach Anspruch 10, wobei die jeweiligen proximalen Abschnitte der feinen Drähte (210, 210a, 210b, 210c, 210d) in elektrischem Kontakt mit dem leitfähigen Einsetzmechanismus (120, 1510) sind, und die jeweiligen distalen Abschnitte der feinen Drähte (210, 210a, 210b, 210c, 210d) innerhalb einer nicht-leitfähigen Barriere zwischen der elektrolytisch ablösbaren Verbindung (110) und der implantierbaren Vorrichtung (130, 1520) angeordnet sind.

12. Baugruppe (100, 200, 300, 400, 500, 800, 1200, 1500, 1700, 1800) nach einem der Ansprüche 1 bis 4, wobei ein jeweiliger Durchmesser jedes der feinen Drähte (210, 210a, 210b, 210c, 210d) zwischen 10 % und 50 % eines Durchmessers eines Platindrahtes beträgt, der verwendet wird, um die implantierbare Vorrichtung (130, 1520) zu bilden.

13. Baugruppe (100, 200, 300, 400, 500, 800, 1200, 1500, 1700, 1800) nach Anspruch 12, wobei ein Durchmesser jedes feinen Drahtes zwischen 0,000254 cm (0,0001 Zoll) und 0,00635 cm (0,0025 Zoll) beträgt und ein Durchmesser des Platindrahtes, der die implantierbare Vorrichtung (130,1520) bildet, zwischen 0,00254 cm (0,001 Zoll) und 0,0127 cm (0,005 Zoll) beträgt.

14. Baugruppe (100, 200, 300, 400, 500, 800, 1200, 1500, 1700, 1800) nach Anspruch 12, wobei die implantierbare Vorrichtung (130) eine gefäßverschließende Spule (1520) umfasst, und wobei ein Durchmesser jedes feinen Drahtes zwischen 1 % und 10 % eines inneren Durchmessers einer ersten Form der Spule (1520) beträgt, und wobei ein Durchmesser jedes feinen Drahtes zwischen 1 % und 5 % eines äußeren Durchmessers einer ersten Form der Spule (1520) beträgt.

## Revendications

1. Ensemble (100, 200, 300, 400, 500, 800, 1200, 1500, 1700, 1800) comprenant :
un dispositif implantable (130, 1520) ;
un mécanisme de déploiement conducteur (120, 1510) pour délivrer le dispositif implantable ; et
une jonction détachable de façon électrolytique (110) qui relie le dispositif implantable (130, 1520) au mécanisme de déploiement conducteur (120, 1510), **caractérisé en ce que** la jonction détachable de façon électrolytique (110) comprend une pluralité de câbles minces (210, 210a, 210b, 210c, 210d) détachables du dispositif implantable (130, 1520) par transmission de courant électrique (2020) à travers ceux-ci.

2. Ensemble (100, 200, 300, 400, 500, 800, 1200, 1500, 1700, 1800) selon la revendication 1, les câbles minces (210, 210a, 210b, 210c, 210d) étant configurés pour être simultanément électrolysés par un courant électrique (2020) appliqué au mécanisme de déploiement conducteur (120, 1510).

3. Ensemble (100, 200, 300, 400, 500, 800, 1200, 1500, 1700, 1800) selon les revendications 1 ou 2, les câbles minces (210, 210a, 210b, 210c, 210d) comprenant de l'acier inoxydable.

4. Ensemble (100, 200, 300, 400, 500, 800, 1200, 1500, 1700, 1800) selon l'une quelconque des revendications 1 à 3, la pluralité des câbles minces (210, 210a, 210b, 210c, 210d) comprenant deux à dix câbles minces.

5. Ensemble (100, 200, 300, 400, 500, 800, 1200, 1500, 1700, 1800) selon l'une quelconque des revendications 1 à 4, chacun des câbles minces (210, 210a, 210b, 210c, 210d) ayant un diamètre compris entre 0,0000254 cm (0,00001 pouce) et 0,002286 cm (0,0009 pouce).

6. Ensemble (100, 200, 300, 400, 500, 800, 1200, 1500, 1700, 1800) selon l'une quelconque des revendications 1 à 4, chacun des câbles minces (210, 210a, 210b, 210c, 210d) ayant une aire de surface comprise entre 5,03 mm carré (.00000000078 pouce carré) et 0,00323 mm carré (.000005 pouce carré).

7. Ensemble (100, 200, 300, 400, 500, 800, 1200, 1500, 1700, 1800) selon l'une quelconque des revendications 1 à 6, la jonction détachable de façon électrolytique (110) comprenant une première pluralité de câbles minces (210), chacun ayant un premier diamètre, et une seconde pluralité de câbles minces (210), chacun ayant un second diamètre différent du premier diamètre.

8. Ensemble (100, 200, 300, 400, 500, 800, 1200, 1500, 1700, 1800) selon l'une quelconque des revendications 1 à 7, les câbles minces (210, 210a, 210b, 210c, 210d) étant enroulés ou tressés (1010).

9. Ensemble (100, 200, 300, 400, 500, 800, 1200, 1500, 1700, 1800) selon l'une quelconque des revendications 1 à 8, les câbles minces (210, 210a, 210b, 210c, 210d) étant raccordés au mécanisme de déploiement conducteur (120, 1510) par une soudure (310) ou un polymère conducteur, et les câbles minces (210, 210a, 210b, 210c, 210d) étant raccordés au dispositif implantable (130, 1520) par un polymère non conducteur.

10. Ensemble (100, 200, 300, 400, 500, 800, 1200, 1500, 1700, 1800) selon l'une quelconque des revendications 1 à 9, dans lequel pour chacun des câbles minces (210, 210a, 210b, 210c, 210d),
une première partie proximale (1410) du câble est nue,
une deuxième partie (1420) adjacente à la première partie (1410) est recouverte d'un revêtement non conducteur (1422),
une troisième partie (1430) adjacente à la deuxième partie (1420) est nue, et
une quatrième partie distale (1440) adjacente à la troisième partie (1430) est recouverte d'un revêtement non conducteur (1422).

11. Ensemble (100, 200, 300, 400, 500, 800, 1200, 1500, 1700, 1800) selon la revendication 10, dans lequel les parties proximales respectives des câbles minces (210, 210a, 210b, 210c, 210d) sont en contact électrique avec le mécanisme de déploiement conducteur (120, 1510), et les parties distales respectives des câbles minces (210, 210a, 210b, 210c, 210d) sont placées à l'intérieur d'une barrière non conductrice entre la jonction détachable de façon électrolytique (110) et le dispositif implantable (130, 1520).

12. Ensemble (100, 200, 300, 400, 500, 800, 1200, 1500, 1700, 1800) selon l'une quelconque des revendications 1 à 4, dans lequel un diamètre respectif de chacun des câbles minces (210, 210a, 210b, 210c, 210d) représente 10 % à 50 % d'un diamètre d'un câble en platine utilisé pour former le dispositif implantable (130, 1520).

13. Ensemble (100, 200, 300, 400, 500, 800, 1200, 1500, 1700, 1800) selon la revendication 12, un diamètre de chaque câble mince étant compris entre 0,000254 cm (0,0001 pouce) et 0,00635 cm (0,0025 pouce), et un diamètre du câble en platine formant le dispositif implantable (130, 1520) étant compris entre 0,00254 cm (0,001 pouce) et 0,0127 cm (0,005 pouce).

14. Ensemble (100, 200, 300, 400, 500, 800, 1200, 1500, 1700, 1800) selon la revendication 12, le dispositif implantable (130) comprenant une bobine vaso-occlusive (1520), et dans lequel un diamètre de chaque câble mince représente 1 % à 10 % d'un diamètre interne d'une forme primaire de la bobine (1520), et dans lequel un diamètre de chaque câble mince représente 1 % à 5 % d'un diamètre externe d'une forme primaire de la bobine (1520).
